# EUROPEAN PATENT SPECIFICATION

(11) **EP 0 611 553 B1**
(45) Date of publication and mention of the grant of the patent: **24.07.1996**
(21) Application number: 94200211.4
(22) Date of filing: 28.01.1994
(51) Int. Cl.: A61B 17/28, B25B 7/08

(54) **Manual implement of synthetic resin, such as forceps for medical use**
Handwerkzeug aus synthetischem Harz, wie zum Beispiel eine medizinische Zange
Outil manuel en résine synthétique tel qu'une pince à usage médical

(30) Priority: 18.02.1993 IT RE930010 U
(43) Date of publication of application: 24.08.1994
(73) Proprietor: C.G.M. S.P.A., I-42015 Corregio (Reggio Emilia) (IT)
(72) Inventor: Parmigiani, Corrado, I-42015 Correggio (Reggio Emilia) (IT)
(74) Representative: Corradini, Corrado

(56) References cited:
- FR-A- 2 024 956
- US-A- 2 305 156
- US-A- 4 813 107

## Description

This invention relates to a manual implement of synthetic resin, such as a forceps for medical use or a like object such as small scissors, comprising two members which mutually intersect in an intermediate region and rotate one relative to the other; of said members, that portion which acts on objects is positioned at one end and that portion which is operated by the hand is positioned at the other end.

In FR-A-2024956, an implement according to the preamble of claim 1 is disclosed.

The purpose of medical forceps is to clamp various objects, for example for squeezing flexible tubes in order to close their passage.

Known medical forceps possess two separate members connected together by a metal rivet which defines a hinge pin. This results in various drawbacks. Firstly, the presence of the metal rivet is a hindrance to the recycling of a discarded forceps for recovering its synthetic resin. In addition, the clinching of the rivet can never be of a constant extent, with the result that the two members are sometimes pressed together too tightly or too loosely.

The object of the present invention is to overcome these and other drawbacks.

The present invention, as characterised in claim 1, provides an implement the construction of which is economical as it is formed by a single moulding operation, it therefore being unnecessary to assemble the two members together (they being formed already assembled). In addition to the preceding features, which are known from FR-A-2024956, the two members are properly guided because one passes through the other, one therefore acting as a guide for the other. They therefore flex to a lesser extent when subjected to force.

In addition the implement according to the invention can be properly recycled as it is constructed completely of synthetic resin.

The invention is described in detail hereinafter with the aid of the accompanying figures, which illustrate one embodiment thereof.

Figure 1 is a view of a forceps for medical use according to the invention.

Figure 2 is a section on the plane II-II of Figure 1.

Figure 3 is a section on the plane III-III of Figure 1.

Figure 4 shows the forceps of Figure 1 in position clamped on a tube.

The implement of the invention consists of two members 2 and 3 which intersect in an intermediate region and are arranged to rotate one relative to the other.

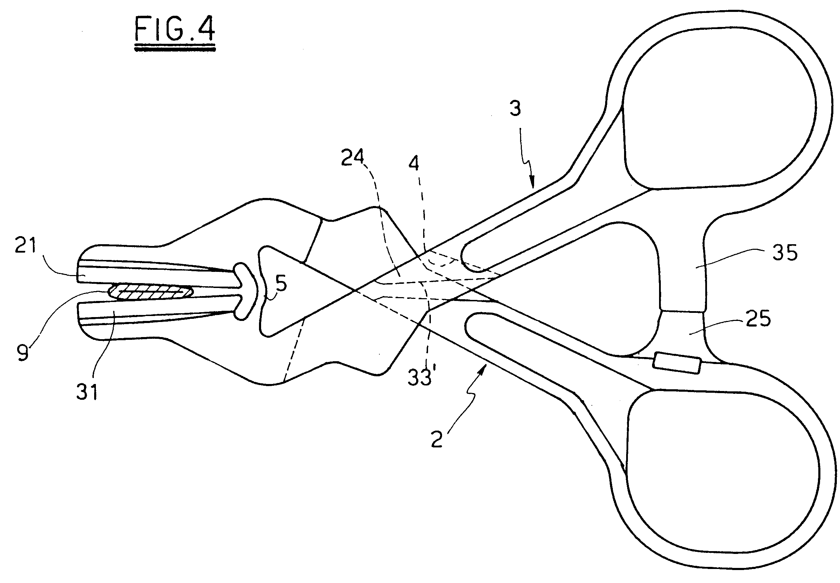

Each member 2, 3 has at one end a portion 21, 31 respectively by which it acts on objects 9 to clamp them (Figure 4). It also has, at its other end, a portion 22, 32 respectively for the operation of the hand. The portions 22 and 32 are for example in the form of eyelets, as in the case of usual scissors.

A flexible strip 5 is provided between the portions 21 and 31 and the region of mutual intersection of the two members 2 and 3 to join the two members 2 and 3 together and define the hinge constraint between them.

Within the thickness of the member 3 there is provided a through slot 4 through which the member 2 passes; the slot 4 has a length (in the direction of the longitudinal central line of the member 3) greater than the width of the member 2 in that region, to enable the two members 2 and 3 to rotate relative to each other.

The slot 4 defines within the member 3 two spaced-apart thin walls 33 which embrace the other member 2 on the two sides.

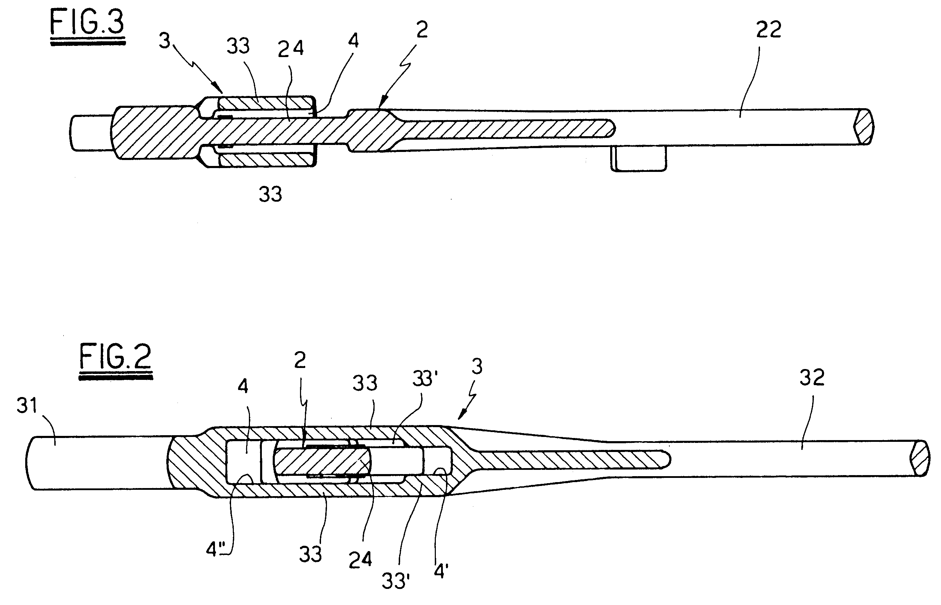

Specifically, the slot 4 has a narrower portion 4' of constant width, in that the distance between the inner surfaces of the walls 33 is constant at that point; said portion 4' is obtained by an inwardly projecting step 33' of triangular plan provided within the thickness of each wall 33 (Figure 2).

The width of the slot portion 4' is substantially equal to the thickness of that portion 24 of the member 2 which moves within the slot 4.

The portion 4' and the portion 24 are engaged with each other under conditions of sliding contact when the forceps is closed (Figure 4). Consequently the two members 2 and 3 are mutually constrained (in the direction of the axis of mutual rotation) in particular when they are in their closed position, this position being that in which most force is exerted on the implement. They are therefore mutually guided and (conveniently) have lesser possibility of bending in the plane within which they rotate.

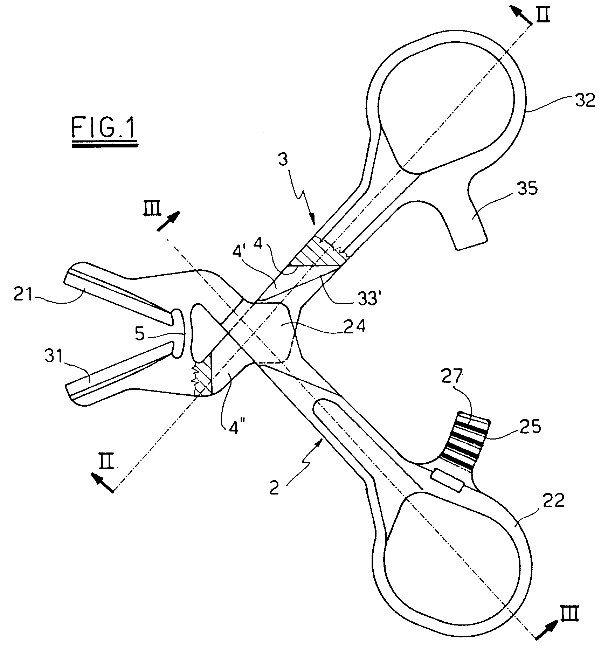

The other portion 4'' of the slot 4 is more displaced towards the strip 5 and has a width substantially greater than the thickness of the portion 24 of the member 2. The slot portion 4'' lies within the region in which the portion 24 intersects the member 3 when the implement is open (Figure 1).

The members 2 and 3 and the strip 5 are formed in one piece from synthetic resin, in particular by a single mould. The greater width of the portion 4'' enables the mould elements required to form the slot 4 and to separate the two members 2 and 3 to be inserted at the moulding stage.

When in use, the two members 2 and 3 are rotated relatively about the intermediate portion of the strip 5, which flexes to hence define a sort of hinge; the strip 5 is made conveniently thin to enable it to flex. When the implement clamps an object between its portions 21 and 32, the action of the hand on the eyelets 22 and 32 subjects the strip to a traction force, the strip 5 consequently being able to conveniently withstand this force even if thin.

On the eyelets 22 and 32 there are provided two tabs 25 and 35 with toothing 27 which, when the implement is in its closed position, mutually engage to maintain the implement in its closed position.

Numerous modifications of a practical and applicational nature can be made to the invention, but without leaving the scope of the claims as claimed hereinafter.

## Claims

1. A manual implement of synthetic resin, such as forceps for medical use, comprising two members (2, 3) which intersect in an intermediate region and are arranged to rotate one relative to the other; of said members (2, 3) that portion (21, 31) which acts on objects being positioned at one of their ends, and that portion (22, 32) which is operated by the hand being positioned at the other end, a flexible strip (5) being provided joining together the two members (2, 3) to define the hinge constraint between the two members (2, 3), and said two members (2, 3) and said strip (5) being formed in one piece from synthetic resin, characterised in that:
- a first said (2) passes through a through slot (4) provided within the thickness of the second said member (3), said slot (4) defining within second member (3) two spaced-apart walls (33) which embrace the first member (2), said slot (4) having a length such as to enable the two members (2,3) to rotate relative to each other.

2. A implement as claimed in claim 1, characterised in that said strip (5) is in an intermediate position between the portions (21, 31) which act on objects and the region of mutual intersection of the two members (2, 3).

3. A implement as claimed in claim 2, characterised in that said slot (4) has a portion (4') of width substantially equal to the thickness of that portion (24) of the first member (2) which moves within the slot (4), said portion (4') of the slot (4) and said portion (24) of the first member (2) being mutually engaged under conditions of sliding contact when the implement is closed.

4. A implement as claimed in claim 3, characterised in that said slot (4) comprises, within the region in which the first and second members (2, 3) intersect when the implement is opened, a second portion (4'') of width substantially greater than the thickness of said portion (24) of the first member (2).

## Patentansprüche

1. Handwerkzeug aus synthetischem Harz, wie etwa Zange für medizinischen Gebrauch, bestehend aus zwei Teilen (2,3), die sich in einem mittleren Bereich kreuzen, und die so angeordnet sind, daß sie relativ zueinander verschwenkbar sind; wobei von diesen Teilen (2,3) der Abschnitt (21,31), der an Gegenständen angreift, an einem ihrer Enden liegt, und der Abschnitt (22,32), der von Hand bewegt wird, an dem anderen Ende liegt, wobei ein biegsamer Steg (5) vorgesehen ist, der die zwei Teile (2,3) gelenkig miteinander verbindet, um das zwischen den zwei Teilen (2,3) erforderliche Scharnier zu bilden, und wobei die zwei Teile (2,3) und der Steg (5) einstückig aus synthetischem Harz gefertigt sind,
**dadurch gekennzeichnet,** daß
ein erstes (2) dieser Teile durch einen Durchgangsschlitz (4) gesteckt ist, der in der Dicke des zweiten Teiles (3) vorgesehen ist, wobei der Schlitz (4) in dem zweiten Teil (3) zwei auf Abstand zueinander stehende Wände (33) bildet, die das erste Teil (2) umfassen, und der Schlitz (4) eine solche Länge hat, daß die zwei Teile (2,3) relativ zueinander verschwenkbar sind.

2. Werkzeug nach Anspruch 1,
**dadurch gekennzeichnet,** daß sich der Steg (5) in einer mittleren Lage zwischen den Abschnitten (21,31), die auf Gegenstände einwirken, und dem Bereich der gegenseitigen Kreuzung der zwei Teile (2,3) befindet.

3. Werkzeug nach Anspruch 2,
**dadurch gekennzeichnet,** daß der Schlitz (4) einen Abschnitt (4') aufweist, mit einer Breite, die im wesentlichen gleich der Dicke jenes Abschnittes (24) des ersten Teiles (2) ist, der sich in dem Schlitz (4) bewegt, wobei der Abschnitt (4') des Schlitzes (4) und der Abschnitt (24) des ersten Teiles (2) unter Gleitkontaktbedingungen in gegenseitigem Eingriff sind, wenn das Werkzeug geschlossen ist.

4. Werkzeug nach Anspruch 3,
**dadurch gekennzeichnet,** daß der Schlitz (4) in dem Bereich, wo sich das erste und das zweite Teil (2,3) kreuzen, wenn das Werkzeug geöffnet ist, einen zweiten Abschnitt (4'') aufweist, mit einer Breite, die im wesentlichen größer ist als die Dicke des Abschnittes (24) des ersten Teiles (2).

## Revendications

1. Outil à main en résine synthétique, tel qu'un forceps pour usage médical, comprenant deux organes (2,3) qui se coupent dans une région intermédiaire et sont disposés de façon à tourner l'un par rapport à l'autre, la partie (21,31) desdits organes (2,3) qui agit sur des objets étant positionnée à une des extrémités desdits organes, et la partie (22,32) qui est actionnée à la main étant positionnée à l'autre extrémité, une bande souple (5) étant prévue qui réunit ensemble les deux organes (2,3) de façon à définir la contrainte d'articulation entre les deux organes (2,3) et desdits deux organes (2,3) et ladite bande (5) étant formés en une seule pièce à partir de résine synthétique, ledit outil étant caractérisé en ce que :
- le premier dit organe (2) passe à travers une encoche traversante (4) prévue dans l'épaisseur dudit second organe (3), ladite encoche (4) définissant à l'intérieur dudit second organe (3), deux parois (33) espacées l'une de l'autre qui embrassent le premier organe (2), ladite encoche (4) ayant une longueur telle qu'elle permet aux deux organes (2,3) de tourner l'un par rapport à l'autre.

2. Outil, selon la revendication 1, caractérisé en ce que ladite bande (5) est située dans une position intermédiaire entre les parties (21, 31) qui agissent sur les objets et la région d'intersection mutuelle des deux organes (2,3).

3. Outil, selon la revendication 2, caractérisé en ce que ladite encoche (4) a une partie (4') de largeur sensiblement égale à l'épaisseur de cette partie (24) du premier organe (2) qui se déplace à l'intérieur de ladite encoche (4), ladite partie (4)' de l'encoche (4) et ladite partie (24) du premier organe (2) étant engagées mutuellement en condition de contact glissant lorsque l'outil est fermé.

4. Outil, selon la revendication 3, caractérisé en ce que ladite encoche (4) comprend, à l'intérieur de la région dans laquelle se coupent le premier et le second organes, (2,3) lorsque l'outil est ouvert, une seconde partie (4'') de largeur sensiblement supérieure à l'épaisseur de ladite partie (24) dudit premier organe (2).
